# EUROPEAN PATENT SPECIFICATION

(11) **EP 4 340 791 B1**
(45) Date of publication and mention of the grant of the patent: **21.01.2026**
(21) Application number: 22726278.9
(22) Date of filing: 18.05.2022
(51) Int. Cl.: A61F 13/02

(54) **WOUND CLOSURE DEVICE**
WUNDVERSCHLUSSVORRICHTUNG
DISPOSITIF DE FERMETURE DE PLAIE

(30) Priority: 19.05.2021 GB 202107181
(43) Date of publication of application: 27.03.2024
(73) Proprietor: Advanced Medical Solutions Limited, Winsford, Cheshire CW7 3RT (GB)
(72) Inventor: HAYWARD, Alistair, Exeter Devon EX1 3BR (GB)
(74) Representative: Naidu, Anis
(86) International application number: PCT/GB2022/051247
(87) International publication number: WO 2022/243675

(56) References cited:
- WO-A1-2019/119292
- US-A1- 2014 155 791
- US-A1- 2015 320 605
- US-A1- 2015 366 719
- US-B2- 10 687 986

## Description

### Field of the Invention

The present invention relates to a wound closure device for closing a wound and a method of manufacturing the same as well as a kit of parts for closing a wound.

### Background of the Invention

Wound closure devices (e.g. wound closure systems) are well known alternatives to the traditional stapling and suturing techniques frequently utilised in wound closure procedures. One of the major problems associated with the conventional stapling and suturing techniques relates to the necessity to puncture the skin as part of the wound closure procedure. This can cause the patient discomfort and tissue trauma as well as a range of other adverse effects, such as, additional bleeding, scarring, tissue irritation, increased risk of infection and longer healing times.

Wound closure devices, such as wound closure strips, have been developed to overcome the issues associated with stapling and suturing techniques. These wound closure devices are generally formed from a flexible material strip having an adhesive, typically a pressure sensitive adhesive (PSA), applied to its wound facing surface. In use, the adhesive on the wound facing surface of the strip acts to gently secure the strip to the skin surrounding the wound and, once the wound has been pulled closed, securely holds the wound in a sealed position. The strip generally remains adhered to the surface of the skin and aligned with the wound during the entire healing process. The strip is often porous so as to allow the flow of fluids through the strip in order to aid the healing process. In order to firmly fix the wound strip in place and prevent its movement during its application and the wound healing process, a further adhesive (e.g. a polymerizable adhesive composition used with a suitable initiator) can be applied over the top of the strip. This further adhesive can also forms a protective barrier over the wound thus enhancing the healing process. Such wound closure strips are quick to apply and avoid the need to puncture the skin during the wound closure procedure. These devices also result in less tissue trauma, enhanced cosmetic appearance and discomfort for the patient.

Despite their benefits, the wound closure strips as described above can be difficult for the user to handle during use. This is because the user is required to manually position and apply the strip to the skin surrounding the wound, which generally involves grasping the edge regions of the strip using his / her fingers and thumbs. This often leads to the user inadvertently touching the adhesive disposed on the wound facing surface of the strip (e.g. the underneath side) therefore resulting in the contamination and rubbing away of the adhesive from the wound facing surface during use. This can compromise the sterility of the wound environment and the bonding strength of the wound closure strip to the wound. Moreover, application of such strips generally require a high level of dexterity from the operator, especially where smaller sized wounds and closure strips are utilised.

In recent times, modified wound closure strips have been developed to overcome the handling issues mentioned above. One particular example involves the incorporation of multiple sections of release liners onto the adhesive disposed on the wound facing surface of the wound closure strip. In such examples, the wound closure strip includes a section of release liner which is removed as normal to expose the adhesive on the wound facing surface of the strip but also includes another section of release liner which is not removed from wound facing surface of the strip until the end of the wound closure procedure. The section of the release liner that remains in adhered to the wound facing surface of the strip can be used to control and position the strip during use. Reference is made to US2018/0085260 A1, which describes a wound closure device having a first section of release liner and second section of release liner applied to an adhesive layer on the wound facing surface of the strip prior to use. The first section of release liner is removed to expose a portion of the wound facing surface of the wound closure strip. With the exposed portion, the wound closure strip is positioned on the wound and used to secure the wound in closed position. The user achieves this by using the second section of the release liner as a handle, where the user is able to grasp the second section of release liner using their thumb and fingers on the top side and under side of the wound closure strip. Once the wound closure strip is in place and the wound secured closed, the second section of release liner can be removed from the underside of the strip to expose the remaining the portion of the adhesive on the wound facing surface of the strip. This is then pressed down onto the surface of the skin to fully secure the strip to the wound.

Solutions such as those described in US2018/0085260 A1, however, suffer from drawbacks of their own. For example, when using devices having multiple sections of release liners located on the adhesive on the wound facing surface of the strip, it can be difficult to remove the section of release liner that serves as the handle portion at the end of the procedure. This is because, at the point of removing the remaining section of release liner from the underside of the strip, there is a large proportion of the closure strip that is already adhered to the surface of the skin at the wound site. This makes it difficult to for the user to manoeuvre their fingers and thumb underneath the strip in order to grasp and peel the remaining release liner away. It is therefore common for the user to inadvertently touch the adhesive layer disposed on the wound facing surface of the strip causing wearing away and contamination. Indeed, in most cases the user will in practice tend to attempt to bend the wound closure strip away from the surface of the skin to make it easier to grip and remove the remaining liner. This can cause the position of the wound closure strip to be inadvertently altered, sometimes compromising the degree to which the strip is aligned with and secured to the wound. US2015/320605A1 relates to island-type wound dressings comprising a backing sheet, a layer of pressure-sensitive adhesive on the backing sheet and an island of absorbent material having a smaller area than the backing sheet. WO2019/119292A1 relates to a wound covering structure, and more particularly to a transparent covering structure, which can observe changes and healing conditions of a wound while providing high moisture permeability and comfort to the wound.

It is an object of the present invention to obviate or mitigate one or more of the above disadvantages.

### Summary of the invention

The present inventor has developed a wound closure device for closing a wound. In general terms, the present inventor has established a wound closure device having a wound closing layer (e.g. wound closure strip) which can be easily handled when applied to a wound and, unlike many known wound closure devices, prevents the user from intentionally or inadvertently grasping or touching the adhesive located on a wound facing surface of the device during use.

In a first aspect of the present invention there is provided a wound closure device comprising (i) a wound closing layer comprising a wound facing surface, a non-wound facing surface opposite to the wound facing surface of the wound closing layer and one or more open areas extending through the wound closing layer from the wound facing surface to the non-wound facing surface of the wound closing layer and (ii) an adhesive disposed on the wound facing surface of the wound closing layer and extending through one or more of the open areas to the extent that, in use, a substrate layer positioned adjacent to the non-wound facing surface of the wound closing layer contacts the adhesive extending through one or more of the open areas.

The wound closure device of the present invention includes an adhesive disposed on the wound facing surface of the wound closing layer (e.g. wound closure strip) which extends through one or more of the open areas in the wound closing layer. The adhesive located on the wound facing surface of the wound closing layer allows for the attachment of the wound closing layer to the skin surround the wound. The adhesive also, advantageously, extends through one or more of the open areas formed in the wound closing layer (e.g. strikes-through the open areas formed in the wound closing layer) to the extent that, in use, a substrate layer (e.g. a release liner or alike) positioned adjacent to the non-wound facing surface of the wound closing layer contacts the adhesive extending through one or more of the open areas. This causes the non-wound facing surface of the adhesive to bond to the substrate layer and retain the substrate layer adjacent to the non-wound facing surface of the wound closing layer. By including a "strike-through" adhesive disposed on the wound facing surface of the wound closing layer but which also extends through open area to the non-wound facing surface, the wound closure device of the present invention possesses (i) wound facing side which is capable of firmly attaches to the skin surrounding wound and (ii) non-wound facing side which has the benefit of being tacky. The non-wound facing side of the device can therefore be used together with a substrate layer (e.g. a release liner or alike) that is designed to easily facilitate the convenient handling of the wound closure device during use and reduces the likelihood of the user from touching, and contaminating, the adhesive on the wound facing side of the wound closing device during use.

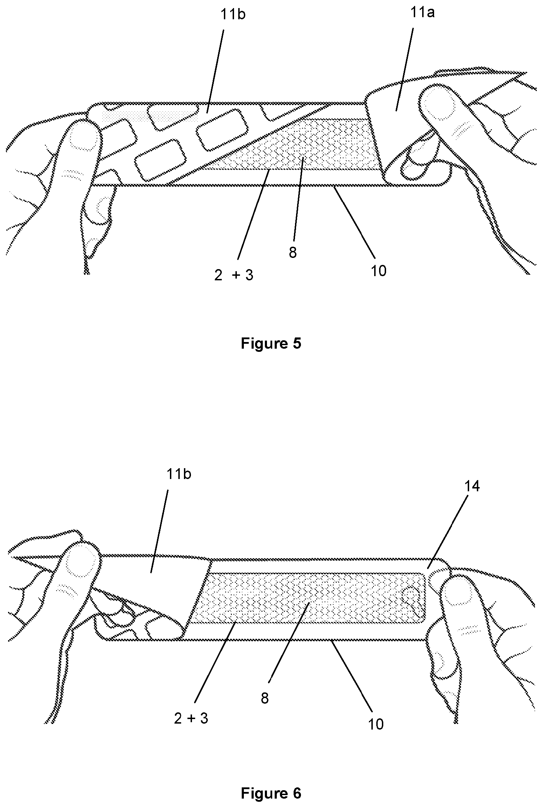

Generally speaking, the adhesive extending through one or more of the open areas of the wound closing layer of the present invention may contact and adhere to a release liner designed to provide a handle with which the user can grasp or grip the device without the need to touch the wound facing side of the adhesive or wound closing layer. As can be seen in exemplary device illustrated in Figures 5-13 described herein, the user is required only to handle the release liner adhered to the non-wound facing surface of the adhesive and wound closing layer in order to manoeuvre and control the position of the device during the wound closing procedure. Once applied and secured to the wound, the release liner can then easily be removed without the need to touch the wound facing surface (i.e. underneath side) of the wound closing layer or adhesive. This requires less skill and dexterity from the user as compared to that required to operate the known devices described above. It will also be appreciated that "strike-through" adhesive removes the requirement to separately apply an additional adhesive to the non-wound facing surface of the wound closing layer when it is so desirable. This results in a more efficient and economical method of manufacture when preparing the wound closured device of the present invention.

In a second aspect of the present invention there is provided a method of manufacturing a wound closure device comprising (i) providing a wound closing layer comprising a wound facing surface, a non-wound facing surface opposite to the wound facing surface and one or more open areas extending through the wound closing layer from the wound facing surface to the non-wound facing surface of the wound closing layer and (ii) disposing an adhesive on the wound facing surface of the wound closing layer and extending through one or more of the open areas to the extent that, in use, a substrate layer positioned adjacent to the non-wound facing surface of the wound closing layer contacts the adhesive extending through one or more of the open areas.

There is also provided a kit of parts for closing a wound comprising a wound closure device according to the first aspect of the present invention or any embodiment thereof, and an applicator comprising a wound closing adhesive.

In embodiments of the first or second aspects of the present invention, the substrate layer is positioned adjacent to the non-wound facing surface of the wound closing layer and contacts the adhesive extending through one or more of the open areas.

In embodiments of the first or second aspects of the present invention, the adhesive extends through the one or more open areas but does not protrude from the non-wound facing surface of the wound closing layer. In other embodiments of the first or second aspects of the present invention, the adhesive extends through the one or more open areas and protrudes from the non-wound facing surface of the wound closing layer but without covering the non-wound facing surface of the wound closing layer. In embodiments, the adhesive on the wound facing surface of the wound closing layer and extending through one or more of the open areas to the extent that, in use, a substrate layer positioned adjacent to the non-wound facing surface of the wound closing layer contacts the adhesive extending through one or more of the open areas, wherein the substrate layer is in contact with the non-wound facing surface of the wound closing layer. In embodiments, the adhesive on the wound facing surface of the wound closing layer and extending through one or more of the open areas to the extent that, in use, a substrate layer positioned adjacent to the non-wound facing surface of the wound closing layer contacts the adhesive extending through one or more of the open areas, wherein the substrate layer is not in contact with the non-wound facing surface of the wound closing layer.

It will be appreciated that, where the adhesive extends through the one or more open areas and protrudes from the non-wound facing surface of the wound closing layer, the adhesive does not spill out from the one or more open areas onto the non-wound facing surface of the wound closing layer. In this embodiment, the adhesive may contact a substrate layer, wherein the substrate layer is not in contact with the non-wound facing surface of wound closing layer. In some instances, the adhesive may extend only partially along the one or more open areas but to the extent that, in use, the adhesive contacts a substrate later. This may, for example, occur where the substrate layer extends (e.g. sags or is depressed) from the non-wound facing surface of the wound closing layer partially along the one or more open areas towards the wound facing surface of the wound closing layer until contact is made with the adhesive. It will also be appreciated that the adhesive may extend along the one or more open areas only to the point where it is level with the non-wound facing surface of the wound closing layer.

### Wound closing layer

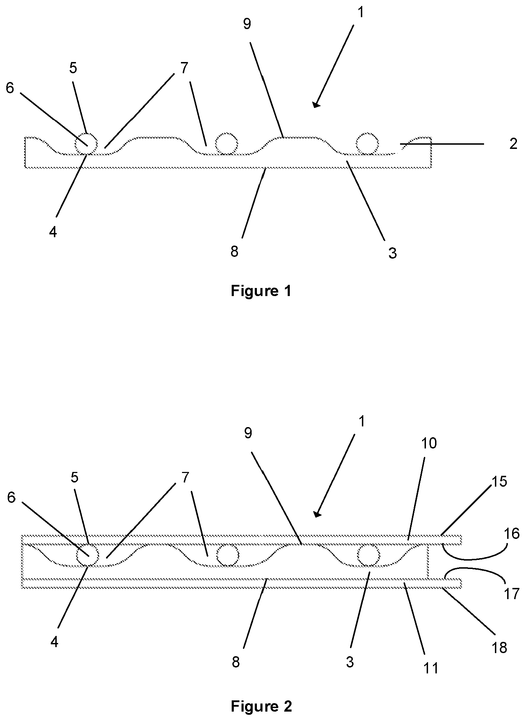

The wound closure device of the present invention comprises a wound closing layer. The wound closing layer has a wound facing surface and a non-wound facing surface opposite to the wound facing surface of the wound closing layer. During use, the wound facing surface of the wound closing layer is located closest to and proximate to the wound opening and the non-wound facing surface is located furthest way from and distal to the wound opening. This is apparent from Figures 1-13 described herein.

The wound closing layer of the present invention includes one or more open areas extending through the wound closing layer from the wound facing surface to the non-wound facing surface of the wound closing layer. In the context of the present invention, the open areas are suitably shaped and dimensioned so as to allow the passage or extending of an adhesive from the wound facing surface to the non-wound facing surface of the wound closing layer. It will be appreciated that the open areas may take the form of an open cell within a foam, a hole / aperture within a perforated film, an open area within a mesh material or alike.

The wound closing layer may be formed from any material possessing the performance characteristics suitable for use in a wound closure procedure and capable of closing a wound. For example, the wound closing layer should be resistance to tearing during throughout the wound closure procedure, in particular, during application onto and removal from a wound. It will be appreciated that the wound closing layer may also be sufficiently flexible and/or elastic so as to conform to the contours of a given wound and accommodate for the movement (e.g. bending or rotation) of the wound during use. The wound closing layer is, preferably, a biologically compatible material which is suitable for use on a tissue during a wound closing procedure. The material is, preferably, approved or regarded as safe for wound closure applications.

In embodiments of the first or second aspects of the present invention, the wound closing layer is a perforated film, mesh, foam (e.g. open celled foam), woven fabric, non-woven fabric or any combination thereof. The wound closing layer may be a mesh or perforated film, preferably, the wound closing layer is a mesh. In further embodiments, the mesh is a mesh fabric, typically, the mesh fabric is a woven mesh fabric.

In further embodiments of the first or second aspect of the present invention, the wound closing layer is formed of a suitable polymer. Typically, the polymer may be selected from the group consisting of polyesters, polyolefins, polyamides, polyethylene, polypropylene, ethylene propylene copolymers, and ethylene butylene copolymers, polyurethanes, polystyrenes, polyvinylchlorides, polylactic acid, polyglycolic acid, polycaprolactone or any combination thereof.

The wound closing layer may be a mesh, typically, a woven mesh fabric. The mesh or woven mesh fabric may be formed of any suitable polymer, preferably, the mesh or woven mesh fabric is formed of a polyester, polyolefin or polyamide. The wound closing layer, in some embodiments, is a polyester woven mesh fabric.

In some embodiments of the first or second aspect of the present invention, the wound closing layer has weight of from about 5 grams per m² to 50 grams per m², from about 10 grams per m² to 50 grams per m², from about 15 grams per m² to 50 grams per m², from about 20 grams per m² to 50 grams per m², from about 25 grams per m² to 50 grams per m², from about 30 grams per m² to 50 grams per m², from about 35 grams per m² to 50 grams per m², from about 40 grams per m² to 50 grams per m² or from about 45 grams per m² to 50 grams per m². In other embodiments, the wound closing layer has weight of from about 5 grams per m² to 45 grams per m², from about 5 grams per m² to 40 grams per m², from about 5 grams per m² to 35 grams per m², from about 5 grams per m² to 30 grams per m², from about 5 grams per m² to 25 grams per m², from about 5 grams per m² to 20 grams per m², from about 5 grams per m² to 15 grams per m² or from about 5 grams per m² to 10 grams per m². The wound closing layer may have a weight of from about 10 grams per m² to 45 grams per m² or the wound closing layer may have a weight of from about 15 grams per m² to 40 grams per m². Typically, the wound closing layer has a weight of from about 5 grams per m² to 30 grams per m², preferably, from about 10 grams per m² to 20 grams per m². The wound closing layer may have a weight of about 15 grams per m².

In embodiments of the first or second aspect of the present invention, the wound closing layer has an open area percentage (%) of from about 10 to about 95. In further embodiments, the wound closing layer has an open area percentage (%) of from about 20 to about 95, from about 30 to about 95, from about 30 to about 95, from about 40 to about 95, from about 50 to about 95, from about 60 to about 95, from about 70 to about 95 or from about 80 to about 95, from about 10 to about 80, from about 10 to about 70, from about 10 to about 60, from about 10 to about 50, from about 10 to about 40, from about 10 to about 30, from about 10 to about 20, from about 20 to about 80, from about 30 to about 70 or from about 40 to about 60. In other embodiments, the wound closing layer has an open area percentage (%) of from about 50 to about 95, typically, from about 70 to about 80. The wound closing layer has an open area percentage (%), preferably, of about 80. It will be appreciated that the open area percentage (%) may reflect how much of the wound closing layer is occupied by open areas. The open area percentage may be varied in order to control the amount of adhesive that is able to extend through (strike-through) the wound closing layer.

According to the first aspect, second aspect or embodiments thereof, the one or more open areas extending through the wound closing layer from the wound facing surface to the non-wound facing surface of the wound closing layer may have a hole area of from about 0.5 mm² to about 5 mm². As used herein, the "hole area" refers to the area occupied by a single one of the "one or more open areas" formed within the wound closing layer of the present invention. In other embodiments, the one or more open areas extending through the wound closing layer from the wound facing surface to the non-wound facing surface of the wound closing layer may have a hole area of from about 0.5 mm² to about 5 mm², from about 0.1 mm² to about 5 mm², from about 2 mm² to about 5 mm², from about 3 mm² to about 5 mm², from about 4 mm² to about 5 mm², from about 0.5 mm² to about 4 mm², from about 0.5 mm² to about 3 mm², from about 0.5 mm² to about 2 mm² or from about 0.5 mm² to about 1 mm². Preferably, the one or more open areas extending through the wound closing layer from the wound facing surface to the non-wound facing surface of the wound closing layer may have a hole area of from about 0.5 mm² to about 2.5 mm² or from about 1 mm² to about 2 mm² or about 1.5 mm².

### Adhesive

According to the first aspect or second aspect or embodiments thereof, the adhesive may be an adhesive layer comprising a wound facing surface and a non-wound facing surface opposite to the wound facing surface of the adhesive layer, wherein the substrate layer positioned adjacent to the non-wound facing surface of the wound closing layer contacts the non-wound facing surface of the adhesive layer. The adhesive may be disposed on the wound facing surface of the wound closing layer as a continuous layer or discontinuous layer. In embodiments, the adhesive may fully or completely cover the wound facing surface of the wound closing layer. In other embodiments, the adhesive may partially cover the wound facing surface of the wound closing layer.

In embodiments where the adhesive partially covers the wound facing surface of the wound closing layer, the percentage (%) coverage of the adhesive on the wound facing surface of the wound closing layer is from about 20% to about 80%, from about 30% to about 80%, from about 40% to about 80%, from about 50% to about 80%, from about 60% to about 80%, from about 70% to about 80%, from about 20% to about 70%, from about 20% to about 60%, from about 20% to about 50%, from about 20% to about 40% or from about 20% to about 30%. In embodiments, the percentage (%) coverage of the adhesive on the wound facing surface of the wound closing layer is from about 20% to about 50%, from about 30% to about 40%, from about 35% to about 45% or from about 35% to about 40%. The coverage of the adhesive on the wound facing surface may be about 35% or 37.5%.

The adhesive layer, in some embodiments, may form a striped pattern or a dotted pattern on the wound facing surface of the wound closing layer. Where the adhesive layer forms a striped pattern, the stripes may be separated by gaps of from about 2mm to about 10mm, preferably, the stripes may be separated by gaps of about 3mm and gaps of about 5mm.

In embodiments of the first and second aspect of the present invention, the non-wound facing surface of the adhesive layer has a peel adhesion value which is less than the peel adhesion value of the wound facing surface of the adhesive layer. Without being bound by theory, in such embodiments, the wound facing surface of the adhesive layer may have a greater surface exposed area than the exposed surface area of the non-wound facing surface of the adhesive layer. This results in the non-wound facing surface of the adhesive layer having a peel adhesion value which is less than the peel adhesion value of the wound facing surface of the adhesive layer.

In some embodiments, the peel adhesion value of the non-wound facing surface of the adhesive layer is from about 0.1 N/25mm to about 5.0 N/25mm, from about 0.1 N/25mm to about 4.0 N/25mm, from about 0.1 N/25mm to about 3.0 N/25mm, from about 0.5 N/25mm to about 5.0 N/25mm, from about 1.0 N/25mm to about 5.0 N/25mm, from about 2.0 N/25mm to about 5.0 N/25mm, from about 3.0 N/25mm to about 5.0 N/25mm or from about 4.0 N/25mm to about 5.0 N/25mm. In other embodiments, the peel adhesion value of the non-wound facing surface of the adhesive layer is from about 0.1 N/25mm to about 2.0 N/25mm, from about 0.2 N/25mm to about 2.0 N/25mm, from about 0.4 N/25mm to about 2.0 N/25mm, from about 0.6 N/25mm to about 2.0 N/25mm, from about 0.8 N/25mm to about 2.0 N/25mm, from about 1.0 N/25mm to about 2.0 N/25mm, from about 1.2 N/25mm to about 2.0 N/25mm, from about 1.4 N/25mm to about 2.0 N/25mm, from about 1.6 N/25mm to about 2.0 N/25mm, from about 1.8 N/25mm to about 2.0 N/25mm, from about 0.1 N/25mm to about 1.8 N/25mm, from about 0.1 N/25mm to about 1.6 N/25mm, from about 0.1 N/25mm to about 1.4 N/25mm, from about 0.1 N/25mm to about 1.6 N/25mm, from about 0.1 N/25mm to about 1.4 N/25mm, from about 0.1 N/25mm to about 1.2 N/25mm, from about 0.1 N/25mm to about 1.0 N/25mm, from about 0.1 N/25mm to about 0.8 N/25mm, from about 0.1 N/25mm to about 0.6 N/25mm, from about 0.1 N/25mm to about 0.4 N/25mm or from about 0.1 N/25mm to about 0.2 N/25mm. In a further embodiment, the peel adhesion value of the non-wound facing surface of the adhesive layer is from about, 0.5 N/25mm to about 1.0 N/25mm. In a preferred embodiment, the peel adhesion value of the non-wound facing surface of the adhesive layer is from about 0.8 N/25mm to about 1.0 N/25mm. The peel adhesion value of the non-wound facing surface of the adhesive layer may be about 0.9 or about 0.89.

In embodiments, the peel adhesion value of the wound facing surface of the adhesive layer is from about 2.5 N/25mm to about 10 N/25mm, from about 4 N/25mm to about 10 N/25mm, from about 6 N/25mm to about 10 N/25mm, from about 8 N/25mm to about 10 N/25mm, from about 2.5 N/25mm to about 8 N/25mm, from about 2.5 N/25mm to about 6 N/25mm or from about 2.5 N/25mm to about 4 N/25mm. In other embodiments, the peel adhesion value of the wound facing surface of the adhesive layer is from about 4 N/25mm to about 8 N/25mm or about 4 N/25mm to about 6 N/25mm. In further embodiments, the peel adhesion value of the wound facing surface of the adhesive layer is from about 2.5 N/25mm to about 5.0 N/25mm or about 4 N/25mm to about 5.0 N/25mm. The peel adhesion value of the wound facing surface of the adhesive layer may be about 4.5 or about 4.29.

In embodiments, the adhesive is disposed (i.e. applied to the wound facing surface of the wound closing layer at a coat weight of from) at a coat weight of from about 5 grams per m² to about 100 grams per m², from about 10 grams per m² to about 100 grams per m², from about 20 grams per m² to about 100 grams per m², from about 30 grams per m² to about 100 grams per m², from about 40 grams per m² to about 100 grams per m², from about 50 grams per m² to about 100 grams per m², from about 60 grams per m² to about 100 grams per m², from about 70 grams per m² to about 100 grams per m², from about 80 grams per m² to about 100 grams per m², from about 90 grams per m² to about 100 grams per m², from about 10 grams per m² to about 90 grams per m², from about 10 grams per m² to about 80 grams per m², from about 10 grams per m² to about 70 grams per m², from about 10 grams per m² to about 60 grams per m², from about 10 grams per m² to about 50 grams per m², from about 10 grams per m² to about 40 grams per m², from about 10 grams per m² to about 30 grams per m² from about 10 grams per m² to about 20 grams per m², from about 20 grams per m² to about 90 grams per m², from about 30 grams per m² to about 80 grams per m², from about 40 grams per m² to about 70 grams per m² or from about 50 grams per m² to about 60 grams per m². In some embodiments, the adhesive is disposed at a coat weight of from about 10 grams per m² to about 30 grams per m², the adhesive has coat weight of from about 10 grams per m² to about 20 grams per m² or the adhesive has coat weight of from about 30 grams per m² to about 50 grams per m². Preferably, the adhesive is disposed at a coat weight of from about 20 grams per m², about 30 grams per m² or about 40 grams per m². In some instances, the adhesive is disposed at a coat weight of from about 35 grams per m². The adhesive may preferably be disposed at a coat weight of from about 20 grams per m².

According to the first and second aspects of the present invention or embodiments thereof, the adhesive may be any suitable adhesive with sufficient tack to adhere to the wound facing surface of the wound closing layer and the surface of a suitable release liner. The adhesive may be any suitable adhesive with sufficient tack to provide an acceptable adhesion to the skin in use. In embodiments, the adhesive comprises a pressure sensitive adhesive. In some embodiments, the adhesive comprises an acrylic based pressure sensitive adhesives or silicone pressure sensitive adhesives or mixture thereof. In some embodiments, the adhesive comprises an acrylic based pressure sensitive adhesive. It will be appreciated that the acrylic based pressure sensitive adhesive may include polymers formed from polymerization of at least one alkyl acrylate monomer or methacrylate, an unsaturated carboxylic acid and optionally a vinyl lactam. Examples of suitable alkyl acrylate or methacrylate esters include, but are not limited to, butyl acrylate, ethyl acrylate, 2-ethylhexyl acrylate, isooctyl acrylate, isononyl acrylate, isodecyl acrylate, methyl acrylate, methylbutyl acrylate, 4-methyl-2-pentyl acrylate, sec-butyl acrylate, ethyl methacrylate, isodecyl methacrylate, methyl methacrylate, and the like, and mixtures thereof. Examples of suitable ethylenically unsaturated carboxylic acids include, but are not limited to, acrylic acid, methacrylic acid, fumaric acid, itaconic acid, and the like, and mixtures thereof.

### Release liners

According to the first aspect and second aspect of the present invention or embodiments thereof, the substrate layer is a first release liner and there is a second release liner in contact with the wound facing surface of the adhesive layer.

It will be appreciated that the first release liner may be any suitable backing or release material used to contact the non-wound facing surface of the adhesive layer and cover the non-wound facing surface of the wound closing layer when positioned adjacent to the wound closing layer. The first release liner has a wound facing surface and a non-wound facing surface opposite to the wound facing surface. The first release liner may be, for example, paper, plastic or alike. In some embodiments, the first release liner is a polyethylene release liner.

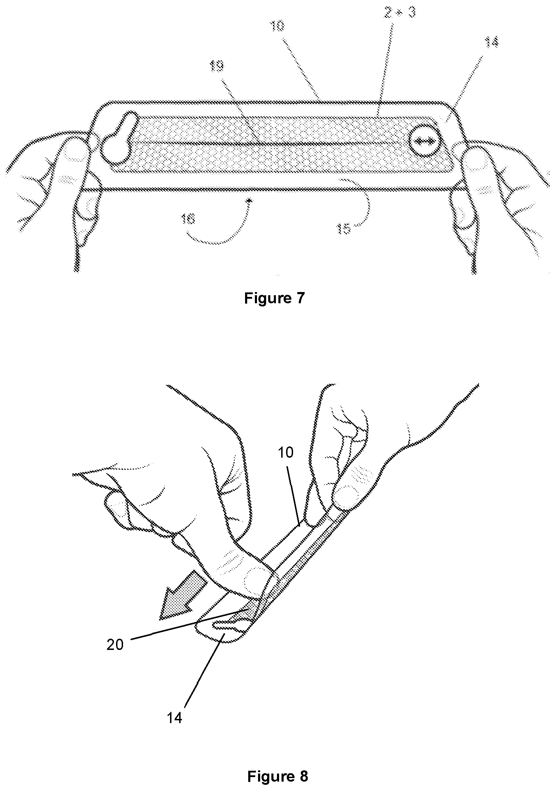

In some embodiments, the first release liner is clear or transparent such that, in use, the user is able to see through the first release liner in order to position and align the mesh-adhesive layer with the wound, as depicted in Figure 7.

In embodiments, when the first release liner is adhered to the non-wound facing surface of the adhesive layer, the first release liner forms a border region around the periphery of the wound closing layer and the adhesive layer. In use, this border region serves as a handle portion which avoids the need for the user to touch the wound facing side of the adhesive layer (i.e. the adhesive on the underside) during use (see Figure 7 and border region 14). In some embodiments, the first release liner may form at least one handle portion which, when the first release liner is adhered to the non-wound facing surface of the adhesive layer, is positioned at the periphery of the wound closing layer and the adhesive layer so as to prevent the user from touching the wound facing surface of the adhesive layer during use. For example, the handle portion may be in the form of at least one tab or flap which may be grasped on the wound facing and/or non-wound facing surfaces during use.

The second release liner may be any suitable backing or release material used to contact and cover the wound facing surface of the adhesive layer when positioned adjacent to wound facing adhesive layer. The second release liner has a wound facing surface and a non-wound facing surface opposite to the wound facing surface. The second release liner may be, for example, paper, plastic or alike. In some embodiments, the second release liner is a glassine release liner, clay coated release liner, PE-coated release liner. Preferably, the second release liner is a glassine release liner.

According to the first and second aspects of the present invention or embodiments thereof, the peel adhesion force (or peel force) required to remove the first release liner from the non-wound facing surface of the adhesive layer is greater than the peel adhesion force (or peel force) required to remove the second release liner from the wound facing surface of the adhesive layer. In such embodiments, the user is able to remove the second release liner from the wound facing surface of the adhesive layer whilst ensuring that the non-wound facing surface of the adhesive layer (strike-through side) remains in contact with and adhered to the wound facing surface of the first release liner during a wound closing procedure. This is made apparent from Figures 5-7 described herein.

In embodiments, the peel adhesion force required to remove the first release liner from the non-wound facing surface of the adhesive layer is from about 0.5 N/40mm to about 10 N/40mm, from about 0.5 N/40mm to about 5 N/40mm, from about 0.5 N/40mm to about 2 N/40mm or from about 0.5 N/40mm to about 1 N/40mm. In embodiments, the peel adhesion force required to remove the first release liner from the non-wound facing surface of the adhesive layer is from about 0.5 N/40mm to about 3 N/40mm, 1 N/40mm to about 3 N/40mm or 2 N/40mm to about 3 N/40mm. In embodiments, the peel adhesion force required to remove the first release liner from the non-wound facing surface of the adhesive layer is from about 2 N/40mm to about 2.5 N/40mm.

In some embodiments, the peel adhesion force required to remove the second release liner from the wound facing surface of the adhesive layer is from about 0.05 N/40mm to about 5 N/40mm, from about 0.05 N/40mm to about 2 N/40mm, from about 0.05 N/40mm to about 1 N/40mm, from about 0.05 N/40mm to about 0.5 N/40mm, from about 0.05 N/40mm to about 0.2 N/40mm. In embodiments, the peel adhesion force required to remove the second release liner from the wound facing surface of the adhesive layer is from about 0.1 N/40mm to about 0.2 N/40mm or about 0.15 N/40mm.

According to some embodiments of the first aspect or second aspect of the present invention, the peel adhesion force required to remove the first release liner from the non-wound facing surface of the adhesive layer is from about 0.5 N/40mm to about 3 N/40mm, from about 1 N/40mm to about 3 N/40mm or from about 2 N/40mm to about 3 N/40mm, and the peel adhesion force required to remove the second release liner from the wound facing surface of the adhesive layer is from about 0.05 N/40mm to about 0.5 N/40mm, from about 0.05 N/40mm to about 0.2 N/40mm. In other embodiments, the peel adhesion force required to remove the first release liner from the non-wound facing surface of the adhesive layer from about 2 N/40mm to about 2.5 N/40mm, and the peel adhesion force required to remove the second release liner from the wound facing surface of the adhesive layer is from about is from about 0.1 N/40mm to about 0.2 N/40mm or about 0.15 N/40mm.

In embodiments where the non-wound facing surface of the adhesive layer has a peel adhesion value which is less than the peel adhesion value of the wound facing surface of the adhesive layer, it will be appreciated that the material properties of the first and second release liners can be selected to ensure that the peel adhesion force required to remove the first release liner from the non-wound facing surface of the adhesive layer is greater than the peel adhesion force required to remove the second release liner from the wound facing surface of the adhesive layer. For example, where the non-wound facing surface of the adhesive layer has a peel adhesion value which is less than the peel adhesion value of the wound facing surface of the adhesive layer, the second release liner may include a coating on its non-wound facing surface which reduces its adhesion strength to the wound facing surface to the adhesive layer to the extent that, in use, the peel adhesion force required to remove the first release liner from the non-wound facing surface of the adhesive layer is greater than the peel adhesion force required to remove the second release liner from the wound facing surface of the adhesive layer. In some embodiments, the second release liner may include a silicone coating on its non-wound facing surface. In some embodiments, the first release liner may include a silicone coating on its wound facing surface and the second release liner may include a silicone coating on its non-wound facing surface such that the relative amounts of the silicone coatings ensure that the peel adhesion force required to remove the first release liner from the non-wound facing surface of the adhesive layer, in use, is greater than the peel adhesion force required to remove the second release liner from the wound facing surface of the adhesive layer. In such embodiments, the second release liner may include a silicone coating on its non-wound facing surface that has a greater coat weight than the silicone coating on the wound facing surface of the first release liner. In these embodiments, the silicone coating on the non-wound facing surface of the second release liner has a coat weight of from about 0.1 grams per m² to about 5 grams per m². Preferably, the silicone coating on the non-wound facing surface of the second release liner has a coat weight of from about 0.1 grams per m² to about 2 grams per m². In other embodiments, a greater percentage surface area of the non-wound facing surface of the second release liner is covered by the silicone coating as compared to the percentage surface area of the wound facing surface of the first release liner covered by the silicone coating.

According to the first and second aspects of the present invention or embodiments thereof, the first release liner includes a tab configured such that, in use, the first release liner is removed from the non-wound facing surface of the adhesive layer by pulling the tab. In such embodiments, the tab is located on the non-wound facing surface of the first release liner. The tab may be operable to be pulled from a central location of the first release liner and towards a longitudinal edge of the first release liner. In some embodiments, the first release liner includes a weakened line (e.g. perforated line) which, when the tab is pulled away from the non-wound facing surface of the adhesive layer during removal, the first release liner tears or breaks along the weakened line. In other embodiments, the first release liner includes a fully broken line (e.g. fully cut line). In such embodiments, the fully broken line allows the user to stretch the wound closing layer when applying the device to the wound. The fully broken line allows for the flexing and/or tensioning of the wound closing layer whilst the first release liner is still attached.

In embodiments according to the first and second aspects of the present invention, the second release liner includes a cut (or perforation) formed diagonally down the middle section of the second release liner. This creates two separate halves to the second release liner. During operation, the user bends the wound closure device in order to cause the second release liner to deform outwardly and the first release liner to deform inwardly. This results in the halves of the second release liner separating or dividing along the cut to allow the user to grasp and remove the liner *via* a peeling action.

The second aspect of the present invention relates to a method of manufacturing a wound closure device comprising (i) providing a wound closing layer comprising a wound facing surface, a non-wound facing surface opposite to the wound facing surface and one or more open areas extending through the wound closing layer from the wound facing surface to the non-wound facing surface of the wound closing layer and (ii) disposing an adhesive on the wound facing surface of the wound closing layer and extending through one or more of the open areas to the extent that, in use, a substrate layer positioned adjacent to the non-wound facing surface of the wound closing layer contacts the adhesive extending through one or more of the open areas.

In embodiments of the second aspect of the present invention, the disposing comprises compressing the wound closing layer and the adhesive together at a suitable compression pressure so as to cause the adhesive to extend through one or more of the open areas to the extent that, in use, a substrate layer positioned adjacent to the non-wound facing surface of the wound closing layer contacts the adhesive extending through one or more of the open areas. In a preferred embodiment, the compressing the wound closing layer and the adhesive together causes the adhesive to extend through the one or more open areas but does not protrude from the non-wound facing surface of the wound closing layer. In some embodiments, the compressing the wound closing layer and the adhesive together causes the adhesive to extend through the one or more open areas and protrude from the non-wound facing surface of the wound closing layer but without causing the adhesive to cover the non-wound facing surface of the wound closing layer. It will be appreciated that the compressing conditions (e.g. pressure and time) required to cause the adhesive to extend through one or more of the open areas to the extent that, in use, a substrate layer positioned adjacent to the non-wound facing surface of the wound closing layer contacts the adhesive extending through one or more of the open areas will vary depending on the material properties of the wound closing layer and the adhesive used.

In some embodiments of the second aspect of the present invention, the compressing is performed using any suitable means for compressing wound closing layer and the adhesive together. For example, the compressing may be performed using nip point roller to compress the wound closing layer and the adhesive together.

In some embodiments of the second aspect of the present invention, the compressing is performed at a pressure of from about 20 Psi to about 40 Psi, from about 25 Psi to about 40 Psi, from about 30 Psi to about 40 Psi, from about 30 Psi to about 40 Psi, from about 20 Psi to about 35 Psi, from about 20 Psi to about 30 Psi or from about 20 Psi to about 25 Psi. In a preferred embodiment, the compressing is performed at a pressure of from about 25 Psi to about 35 Psi or about 30 Psi.

In certain embodiments of the second aspect of the present invention, prior to the step of disposing, the following steps are performed (i) providing a carrier layer comprising a wound facing surface and a non-wound facing surface opposite to the wound facing surface of the carrier layer and (ii) applying an adhesive-solvent mixture onto the non-wound facing surface of the carrier layer and removing the solvent to form the adhesive. It will be appreciated that the carrier layer may be a sacrificial carrier layer (e.g. sacrificial paper liner) which is removed from the wound facing surface of the adhesive and replaced with a second release liner as described herein before use.

In some embodiments, the removing the solvent comprises drying at a temperature of from about 50°C to about 150°C to form the adhesive. It will be appreciated that the drying temperature may vary depending on the properties of the adhesive-solvent mixture used.

In embodiments of the second aspect of the present invention, a first release liner (i.e. a substrate layer) as described herein is applied to the non-wound facing surface of the adhesive.

According to the first and second aspects of the present invention or embodiments thereof, the wound closure device may include one or more auxiliary layers. Such auxiliary layers may include further release liners forming a packaging system for the wound closure device.

According to the second aspect of the present invention or embodiments thereof, the adhesive is an adhesive layer comprising a wound facing surface and a non-wound facing surface opposite to the wound facing surface of the adhesive layer, wherein the non-wound facing surface of the carrier layer is in contact with the wound facing surface of the adhesive layer, and the disposing an adhesive comprises contacting the wound facing surface of the wound closing layer with the non-wound facing surface of the adhesive layer. In such embodiments, the adhesive is disposed at a coat weight of from about 20 grams per m², about 30 grams per m² or about 40 grams per m². In some instances, the adhesive is disposed at a coat weight of from about 35 grams per m². The adhesive may preferably be disposed at a coat weight of from about 20 grams per m².

The kit of parts for closing a wound, as described herein, comprising a wound closure device according to the first aspect of the present invention or any embodiment thereof, and an applicator comprising a wound closing adhesive. In such embodiments, the wound closing adhesive may be a polymerizable adhesive composition and optionally an accelerant and/or initiator. In certain embodiments, the polymerizable adhesive composition is different to the adhesive adhered to the wound closing layer.

In embodiments, the kit further comprises additional components. The different components or groups of components may be sterilized in separate containers before packaging the components or groups of components within a kit, and thereafter sterilizing the kit.

In some embodiments of the first and second aspects of the present invention, the wound closure device comprises a wound closing layer comprising a wound facing surface, a non-wound facing surface opposite to the wound facing surface of the wound closing layer and one or more open areas extending through the wound closing layer from the wound facing surface to the non-wound facing surface of the wound closing layer; and an adhesive disposed on the wound facing surface of the wound closing layer and extending through one or more of the open areas to the extent that, in use, a substrate layer positioned adjacent to the non-wound facing surface of the wound closing layer contacts the adhesive extending through one or more of the open areas, wherein the substrate layer is positioned adjacent to the non-wound facing surface of the wound closing layer and contacts the adhesive extending through one or more of the open areas, wherein (i) the adhesive extends through the one or more open areas but does not protrude from the non-wound facing surface of the wound closing layer; or (ii) the adhesive extends through the one or more open areas and protrudes from the non-wound facing surface of the wound closing layer but without covering the non-wound facing surface of the wound closing layer, wherein the adhesive is an adhesive layer comprising a wound facing surface and a non-wound facing surface opposite to the wound facing surface of the adhesive layer, wherein the substrate layer positioned adjacent to the non-wound facing surface of the wound closing layer contacts the non-wound facing surface of the adhesive layer, wherein the non-wound facing surface of the adhesive layer has a peel adhesion value which is less than the peel adhesion value of a wound facing surface of the adhesive layer, optionally wherein the peel adhesion value of the non-wound facing surface of the adhesive layer is from about 0.5 N/25mm to about 1.0 N/25mm and the peel adhesion value of the wound facing surface of the adhesive layer is from about 2.5 N/25mm to about 5.0 N/25mm, the wound closing layer has a weight of from about 10 grams per m2 to 20 grams per m2, also the wound closing layer is formed of a polymer selected from the group consisting of polyesters, polyolefins and polyamides, the wound closing layer has an open area percentage (%) of from about 70 to about 95, the wound closing layer is a woven mesh fabric, the adhesive has coat weight of from about 10 grams per m2 to about 30 grams per m2, the adhesive comprises a pressure sensitive adhesive, preferably, an acrylic based pressure sensitive adhesives; optionally wherein the substrate layer is a first release liner and there is a second release liner in contact with the wound facing surface of the adhesive layer; optionally wherein the peel adhesion value required to remove the first release liner from the non-wound facing surface of the adhesive layer is greater than the peel adhesion value required to remove the second release liner from the wound facing surface of the adhesive layer.

### Description of the figures

The present invention will now be described with reference to the following non-limiting examples and figures, which show:
**Figure 1****:** A side or cross-sectional view of a wound closure device according to an embodiment of the invention without release liners.
**Figure 2****:** A side or cross-sectional view of a wound closure device according to an embodiment of the invention with release liners present.
**Figure 3****:** A side or cross-sectional view of a wound closure device according to an embodiment of the invention with a release liner adhered to the non-wound facing surface of the adhesive and positioned adjacent to the non-wound facing surface of the wound closing layer (e.g. mesh).
**Figure 4****:** A perspective view of a wound closure device according to an embodiment of the invention shown wherein the wound closing layer and release liners are depicted in an exploded form.
**Figure 5****:** A plan view of a wound closure device according to an embodiment of the invention shown with a first half of a release liner being removed from the wound facing surface of the adhesive and wound closing layer.
**Figure 6****:** A plan view of a wound closure device according to an embodiment of the invention shown with a second half of a release liner being removed from the wound facing surface of the adhesive and wound closing layer.
**Figure 7****:** A perspective view of a wound closure device according to an embodiment of the invention wherein the device is located and aligned over a wound opening.
**Figure 8****:** A perspective view of a wound closure device according to an embodiment of the invention wherein the wound closure device is applied to an area of skin surrounding the wound opening.
**Figure 9****:** A perspective view of a wound closure device according to an embodiment of the invention wherein the wound closure device is used to close a wound opening.
**Figure 10****:** A perspective view of a wound closure device according to an embodiment of the invention wherein the wound closure device is being fully applied to an area of skin surrounding the wound.
**Figure 11****:** A perspective view of a wound closure device according to an embodiment of the invention depicting the user grasping a tab before removing a release liner from the non-wound facing surface of the adhesive and wound closing layer.
**Figure 12****:** A perspective view of a wound closure device according to an embodiment of the invention wherein a release liner is being removed from the non-wound facing surface of the adhesive and wound closing layer.
**Figure 13****:** A perspective view of a wound closure device according to an embodiment of the invention wherein the user is applying a further adhesive to the closed wound using an applicator device.

### Detailed description of the invention

Referring to Figure 1, there is depicted a wound closure device 1, for closing a wound (not shown) according to a preferred embodiment of the present invention. As described herein, the wound closure device 1 can vary in size and shape depending on the nature and dimensions of the wound to which the device is being applied. It will be appreciated that the materials used to make the wound closure device 1 may also vary depending on the specific requirements of the wound to which the device is being applied.

As shown in Figure 1, the wound closure device 1 includes a wound closing layer 2 and an adhesive 3. The wound closing layer 2 has a wound facing surface 4, on which the adhesive is disposed, as well as a non-wound facing surface 5. The wound closing layer 2 is a woven mesh material containing filaments 6 that form the mesh material. The woven mesh material includes open areas (or pores) 7 which extend from the wound facing surface 4 through to the non-wound facing surface 5 of the mesh wound closing layer 2. As depicted in Figures 1-3, in the form of cross-sectional schematics, adhesive 3 forms an adhesive layer which is disposed on and adhered to the wound facing surface 4 of the mesh wound closing layer 2 but which also extends through the open areas 7 of the mesh wound closing layer 2. The adhesive layer 3 has a wound facing surface 8 and non-wound facing surface 9, as shown. It will be appreciated that the extending of adhesive from the wound facing surface of the wound closing layer to the non-wound facing surface of the wound closing layer may, in some instances, be referred to as "strike-through". In the embodiment depicted in Figures 1-3, the adhesive layer 3 depicted does not protrude from or extend beyond the non-wound facing surface 5 of the mesh wound closing layer 2. It will be appreciated that is some embodiments, the adhesive layer 3 may protrude or extend beyond the non-wound facing surface 5 of the mesh wound closing layer 2 but without covering the the non-wound facing surface 5 of the mesh wound closing layer 2.

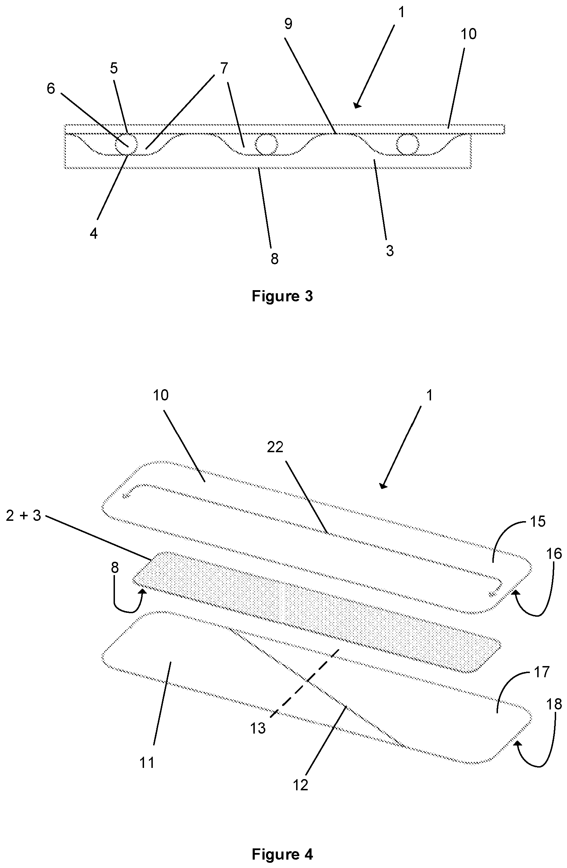

Before use, the wound closure device 1 includes a first release liner 10 in contact with and adhered to the non-wound facing surface 9 of the adhesive layer 3, as shown in Figure 2. The first release liner 10 is adhered to the non-wound facing surface 9 of the adhesive layer 3 *via* its wound facing surface 16 (see Figure 4 also). The first release liner 10 is therefore held adjacent to the non-wound facing surface 5 of the mesh wound closing layer 2. A second release liner 11 is also in contact with and adhered to the wound facing surface 8 of the adhesive layer 3. The second release liner 1 is adhered to the wound facing surface 8 of the adhesive layer 3 *via* its non-wound facing surface 17 (see Figure 4 also). The adhesive layer 3 extends through the open areas 7 of the mesh wound closing layer 2 in order to contact the wound facing surface 16 of the first release liner 10 and retain the first release liner 10 in a position adjacent to the non-wound facing surface 9 of the adhesive layer 3.

For the avoidance of doubt, the mesh wound closing layer 2 and the "strike-through" adhesive layer 3 are depicted together in Figures 4-13 and, where appropriate, are labelled as "2+3". It will be appreciated that when mentioned herein "mesh-adhesive layer" and/or "2+3" refers to the combination of the mesh wound closing layer 2 and adhesive layer 3. The arrangement of the mesh-adhesive layer labelled 2+3 in Figures 4-13 is consistent with that shown in Figures 1-3.

Figure 4 shows a perspective view of the wound closure device 1, before use, in exploded form. As seen in Figure 4, the mesh-adhesive layer 2+3 as well as the first release liner 10 and second release liner 11 are rounded rectangles. In Figure 4, the first release liner 10 and second release liner 11 are sized in such a way that they form a border around the mesh-adhesive layer 2+3 with the mesh-adhesive layer 2+3 located as an island at the centre of the release liners. This is described in more detail with reference to Figures 5-13 below. The first release liner 10 has a wound facing surface 16 and non-wound facing surface 15 whilst the second release liner 11 has a wound facing surface 18 and a non-wound facing surface 17 (as also shown in Figure 2).

During operation, the user removes release liner 11 *via* a peeling action as shown in Figure 5. As seen, the user grasps the wound closure device 1 in one hand, typically between his / her thumb and index finger, whilst pulling away a first half of the release liner 11a with the other hand. As shown in Figure 4, the release liner 11 may include a cut (or perforation) 12 formed diagonally down the middle section of the release liner 11. This creates the two separate halves (11a and 11b) of the release liner 11. When the user bends the wound closure device 1 along the axis 13 (see broken line in Figure 4), by forcing the second release liner 11 to deform outwardly and the first release liner 10 to deform inwardly, the separate halves (11a and 11b) of the second release liner 11 separate along cut 12. This causes the inner edges of the two halves 11a and 11b of the release liner 11 to lift away along cut 12 thus allowing the user to easily grasp the liner 11a before its removal. The user is then able remove the remaining half 11b of the release liner 11 in a similar way, as shown in Figure 6, to fully expose the wound facing surface 8 of the adhesive layer 3. As shown in Figure 6, the user is able to grip a border region 14 of the first release liner 10 so as to avoid touching the wound facing surface 8 of the adhesive layer 3.

It is observed that when the second releaser liner 11 is removed from the wound facing surface 8 of the adhesive layer 3, the mesh-adhesive layer 2+3 remains secured to the first release liner 10. That is, the non-wound facing surface 9 of the adhesive layer 3 remains adhered to the wound facing surface 16 of the first release liner 10. This is possible since the peel adhesion force required to separate the non-wound facing surface 9 of the adhesive layer 3 from the wound facing surface 16 of the first release liner 10 is greater than that of the peel adhesion force required to separate the wound facing surface 8 of the adhesive layer 3 from the non-wound facing surface 17 of the second releaser liner 11. Whilst the relative peel adhesion value of the wound facing surface 8 of the adhesive layer 3 may be greater than the peel adhesion value of the non-wound facing surface 9 of the adhesive layer 3, as shown by the tests described herein, it is possible to control the peel adhesion forces required to separate the first and second release liners by carefully selecting the respective release liners properties. Here, by appropriately selecting the release liner properties, it can be ensured that the peel adhesion force required to separate the non-wound facing surface 9 of the adhesive layer 3 from the wound facing surface 16 of the first release liner 10 is greater than that of the peel adhesion force required to separate the wound facing surface 8 of the adhesive layer 3 from the non-wound facing surface 17 of the second releaser liner 11. One way of achieving this is to use a second release liner 11 which includes a coating (e.g. a silicone coating) on its non-wound facing surface 17 which reduces its adhesion strength with the wound facing surface 8 of the adhesive layer 3. In some instances, the coat weight of this coating may play an important role in controlling the peel adhesion force required to separate the wound facing surface 8 of the adhesive layer 3 from the non-wound facing surface 17 of the second releaser liner 11.

Once the second release liner 11 has been removed to fully expose the wound facing surface 8 of the adhesive layer 3, the user is able to grasp the border region 14 of the first release liner 10. As shown in Figure 7, the user's hands and fingers do not come into contact with the wound facing surface 8 of the adhesive layer 3 and, instead, remain on the border region 14 of the first release liner 10. Figure 3 shows a cross-sectional view of the wound closure device 1 with the second release liner 11 removed prior to the device being applied to the wound.

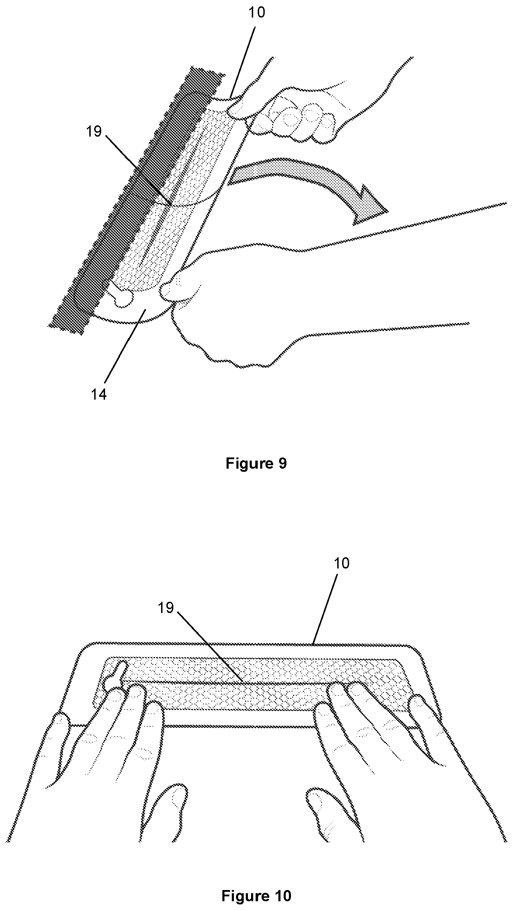

Between Figures 6 and 7, the user inverts the device such that, as shown in Figure 7, the user's thumbs are positioned within the border region 14 but on the non-wound facing surface 15 of the first release liner 10 whilst the user's index fingers are positioned within the border region 14 but on the wound facing surface 16 of the first release liner 10. At this stage, the wound closure device is ready for application to a wound opening 19, as shown in Figure 7. Without needing to touch any part of the mesh-adhesive layer 2+3, the user is able to carefully position the mesh-adhesive layer 2+3 over the wound opening 19. In a preferred embodiment, the first release liner 10 is sufficiently transparent (or clear) to allow the user to see the mesh-adhesive layer 2+3 and wound opening 19 through the liner 10 during use in order to accurately align the mesh-adhesive layer 2+3 with the wound opening 19. Once the user has the device in the desired position, the user carefully applies a portion of the mesh-adhesive layer 2+3 onto an area of skin located at one side of the wound opening 19. The user then adheres the mesh-adhesive layer 2+3 to the skin by applying a pressure to the non-wound facing surface 15 of the first release liner 10 as shown in Figures 8 and 9. The pressure is applied by the user to the non-wound facing surface 15 of the first release liner 10 in the region 20 shown in Figure 8. It will be appreciated that this region may vary in size and shape depending on the nature, size and shape of both the wound opening 19 and the wound closure device 1 itself. The application of pressure to the non-wound facing surface 15 of the first release liner 10 causes the wound facing surface 8 of the adhesive layer 3 to adhere to the surface of the skin. This secures the mesh-adhesive layer 2+3 to an area of skin located at one side of the wound opening 19. The mesh-adhesive layer 2+3 remains untouched by the user.

Once the device is secured to the of skin located on one side of the wound opening 19, as shown in Figures 8 and 9, the user grasps and pulls the border region 14 of the liner 10 in order to skin adhered to the mesh-adhesive layer 2+3 towards the area of skin located on the other wide of the wound opening 19. This action is shown in Figure 9 where the user's direction of pulling is illustrated by the arrow. Once the wound is approximated (i.e. closed), the user then applies the remainder of the exposed mesh-adhesive layer 2+3 to the skin by applying a pressure to the non-wound facing surface 15 of the first release liner 10 as shown in Figure 10. This secures the wound 19 in its closed position. As described above, with reference to Figures 1-10, the user is able to quickly and conveniently handle the wound closure device 1 of the present invention without needing to touch any part of the mesh-adhesive layer 2+3. This is largely facilitated by the liner 10 which is advantageously adhered to the non-wound facing surfaces 9 of the adhesive layer 3. The liner 10 acts as a convenient handle and support structure for the mesh-adhesive layer 2+3 with which to manipulate the device during the wound closure procedure without needing to touch or contact the wound facing surface 8 of the adhesive layer 3.

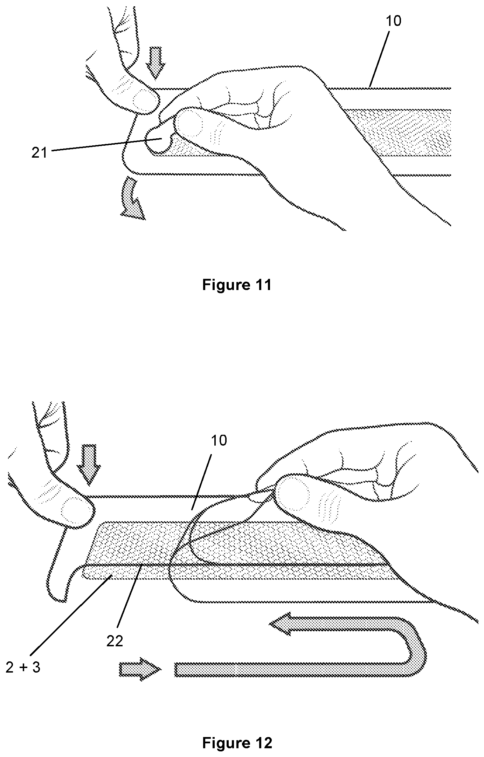

After being secured at the desired position on the closed wound, the user then removes the first release liner 10 as depicted in Figures 11 and 12. In some embodiments, this is achieved using tab 21. Here, the user holds the wound closure device 1 in place using one hand (see straight arrow in Figure 11) whilst lifting tab 21 away from the non-wound facing surface 15 of first release liner 10. The user pulls the tab 21 in a direction starting from a central location of the liner 10 and towards a longitudinal edge of the first release liner 10 (as shown by the curved arrow in Figure 11). This causes the first release liner 10 to tear along a weaken line 22 (e.g. perforated or cut line), shown in Figures 4 and 12, as the user peels the first release liner 10 away from the mesh-adhesive layer 2+3. The first release liner 10 is pulled away and removed from the non-wound facing surfaces, 5, 9 of mesh-adhesive layer 2+3 in a direction as depicted by the arrows in Figure 12.

It can be seen that when the first release liner 10 is removed from the non-wound facing surface 9 of the adhesive layer 3, the mesh-adhesive layer 2+3 remains secured to the skin surrounding the wound. That is, the wound facing surface 8 of the adhesive layer 3 remains adhered to the skin surrounding the wound whilst the first release liner 10 is being removed. This is possible because the peel adhesion force required to separate the wound facing surface 8 of the adhesive layer 3 from the skin surrounding the wound is greater than the peel adhesion force required to separate the non-wound facing surface 9 of the adhesive layer 3 from the wound facing surface 16 of the first release liner 10.

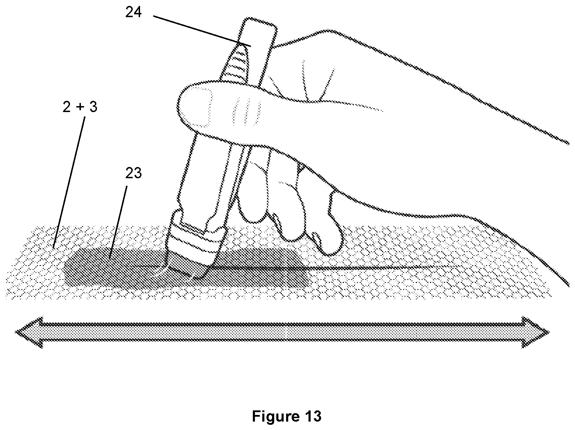

A further adhesive 23 (e.g. polymerizable adhesive + initiator / accelerant) may then be applied to mesh-adhesive layer 2+3 using a suitable applicator device 24 as shown in Figure 13.

### Preparation of the wound closure device

The wound closure device of the present invention may be suitably manufactured according to the exemplary method provided below.
1. A solvent based acrylic pressure sensitive adhesive (purchased from Raleigh Coatings, Product ID: RM0004) was applied to a sacrificial paper release liner at a coat weight of 35 grams per m². The adhesive contains a mixture of heptane, isopropanol, ethyl acetate, toluene and pentandione. The adhesive has the following properties (i) peel adhesion of 36 N/25 mm, (ii) loop tack of 16 N/25 mm² and (iii) shear test of 0.2 hours when tested at 30 grams per m² on 50 µm PET. The adhesive also has a solids content of 49% to 53% and a viscosity of 1750 to 3500 mPas.
2. The sacrificial paper release liner with the layer of pressure sensitive adhesive disposed thereon was then passed through an oven in order to remove solvents by evaporation. The oven contains three zones through which the sacrificial paper liner and adhesive are passed. Zone 1 is set at a temperature of 50°C, zone 2 is set at a temperature of 100°C and zone 3 is set at a temperature of 112°C.
3. A soft net woven mesh (i.e. wound closing layer) purchased from Heathcoat Fabrics was then disposed onto the surface of the pressure sensitive adhesive (i.e. the surface opposite to the adhesive layer surface which is in contact with the sacrificial paper release liner) to form a mesh-adhesive laminate (i.e. mesh-adhesive layer). Material characteristics and properties of the woven mesh are presented in Table 1 below.

**Table 1**

| **Structural Characteristic / Mechanical & Physical Performance** | **Value** |
|---|---|
| Material composition | 100 % polyester |
| Weight | 14 g/m² |
| Course (inch) | 53 |
| Wales (inch) | 17 |
| Open area | ~79 % |
| Hole area | ~1.3 mm² |

4. The mesh-adhesive laminate was then passed through a nip point at a compression pressure of 30 Psi.
5. The laminate was then rolled into a log and slit into logs of smaller width.
6. The sacrificial paper release liner was then then removed and replaced with a paper release liner based on glassine (Supplier: Laufenberg - Product name: KS 900 white, 51B/51B8). The liner is formed with a diagonal split down the middle (as shown in Figures 4-6). The paper release liner was purchased from Laufenberg GmbH. The paper release liner has a grammage of 92 gram per m² and caliper of 80 µm. It will be appreciated that the paper release liner is adhered to the wound facing surface of the adhesive layer whilst the mesh is adhered to the non-wound facing surface of the adhesive layer.
7. A clear polyethylene release liner (base material OPET-film) (Supplier: Laufenberg - Product name: PETP 36 transparent, 51B16) is then laid over the non-wound facing surface of the mesh-adhesive laminate to form the exemplary wound closure device of the present invention. The clear polyethylene release liner has a grammage of 48 - 56 gram per m² and caliper of 34 - 40 µm.

Suitable release liners, mesh and adhesives may be purchased or prepared using conventional methods.

### Experimental Tests

### Measurement of peel adhesion values for the wound closure device

Peel adhesion data for a wound closure device according to the present invention was measured according the method described below. The sample of wound closure device on which these tests were performed was prepared using the method described under the section "Preparation of the wound closure device" described above.

In order to the measure the peel adhesion values for the wound facing surface of the adhesive layer and the non-wound facing surface of the adhesive layer, release liners were removed to provide the wound closure device in the form of the mesh-adhesive layer (as shown in Figure 1). It will be appreciated that the wound facing surface of the adhesive layer on which the following tests were performed corresponds to wound facing surface 8 as described above with reference to Figures 1-13. The non-wound facing surface of the adhesive layer (i.e. strike-through side) on which the following tests were performed corresponds to non-wound facing surface 9 as described above with reference to Figures 1-13.

A sample of the mesh-adhesive layer was cut to 25mm x 150mm using a cutting press and die. A stainless steel plate was cleaned with isopropyl alcohol or acetone. The side of the mesh-adhesive layer to be tested was then adhered to the stainless steel plate. A 1kg roller was passed over the top of the mesh-adhesive layer to ensure the sample was secured. The stainless steel plate was then mounted onto the test rig of a suitable tensometer. A section of the mesh-adhesive layer was clamped in an upper jaw of the tensometer at a 90 degree angle. The upper jaw was moved up, peeling the mesh-adhesive layer away from the stainless steel plate at a rate of 5 mm/s. The maximum and mean forces taken to remove mesh-adhesive layer were measured by the tensometer. Ten separate measurements were taken for both the wound facing surface and the non-wound facing surface of the mesh-adhesive layer. The mean values for both surfaces of the mesh-adhesive layer are shown in Table 2.

**Table 2**

| | **N° of measurements** | **Peel adhesion - Mean value (N/25mm)** |
|---|---|---|
| Wound facing surface of adhesive layer | 10 | 4.29 |
| Non-wound facing surface (strike-through side) of adhesive layer | 10 | 0.89 |

As shown by the experimental test data in Table 2, the wound facing surface of the adhesive layer forming part of the wound closure device (corresponding to wound facing surface 8 in the Figures 1-13) possesses a greater mean peel adhesion value of 4.29 N/25mm as compared to the non-wound facing surface of the adhesive layer forming part of the wound closure device or strike-through side (corresponding to the non-wound facing surface 9 in Figures 1-13) which is 0.89 N/25mm. In use, this difference in peel adhesion values between the wound facing side and strike-through side beneficially allows for the mesh-adhesive layer (corresponding to mesh-adhesive layer 2+3 in Figures 1-13) to remain adhered to the skin surrounding the wound whilst a liner adhered to the strike-through side is being removed.

### Measurement of peel adhesion value for the removal of the paper release liner (i.e. second release liner) from the wound facing surface of the adhesive layer forming part of the wound closure device of the present invention

The sample of wound closure device on which the following tests were performed was prepared using the method described under the section "Preparation of the wound closure device" described above. It will be appreciated that the wound facing surface of the adhesive layer on which following tests were performed corresponds to wound facing surface 8 as described above with reference to Figures 1-13. The paper release liner on which following tests were performed corresponds to second release liner 11 as described above with reference to Figures 1-13. The clear polyester release liner on which following tests were performed corresponds to second release liner 10 as described above with reference to Figures 1-13.

A sample of the wound closure device was cut to 70mm x 125mm (the width of the mesh-adhesive layer located between liners was 40mm) using a cutting press and die. The paper release liner was clamped in the lower jaws of a tensometer using an uncut 70mm side. The clear polyester liner was then clamped in the upper jaws of the tensometer at a 180 degree angle using the same 70mm side. The jaws were moved in opposing directions at a rate of 5 mm/s causing the liners to be pulled in opposite directions. The maximum and mean peel adhesion values were measured by the tensometer. In order for the test to be valid, the mesh-adhesive layer needed to remain adhered to the clear polyester liner with the paper release liner being removed. Measurements were repeated with 56 sample. The data is shown in Table 3 below.

### Measurement of peel adhesion value for removal of clear polyester release liner (i.e. first release liner) from the non-wound facing surface of the adhesive layer (i.e. strike-through side) forming part of the wound closure device of the present invention

The peel adhesion data for the removal of the clear polyester release liner was obtained using the method described below starting from the sample obtained after the removal of the paper release liner described in the test above.

A stainless steel plate was cleaned with isopropyl alcohol or acetone. A sample with the clear polyester release liner still in place was adhered to the stainless steel plate using the surface that was previously attached to the paper liner (i.e. the wound facing surface of the adhesive layer). The piece of stainless steel was sized such that once the sample was adhered to the steel, there was an extra 10mm of steel around the mesh in each direction. A 1kg roller was passed over the top of the clear polyester liner three times to ensure the sample was firmly secured to the steel plate. The stainless steel plate was then mounted onto the test rig of a tensometer. A section of the clear polyester liner was clamped in the upper jaw of the tensometer at a 90 degree angle using the 70mm side of the sample. The upper jaw was moved up, peeling the clear polyester liner away from the non-wound facing surface of the adhesive layer and the stainless steel plate at a rate of 5 mm/s. The force taken to remove the clear polyester liner was measured by the tensometer. Measurements were repeated with 56 samples. The data is shown in Table 3.

**Table 3**

| | **N° of measurements** | **Peel adhesion - Mean value (N/40mm)** |
|---|---|---|
| Removal of paper release liner | 56 | 0.14 |
| Removal of clear polyester liner | 56 | 2.21 |

As shown by the experimental test data in Table 3, peel adhesion force for the removal of the paper release liner (i.e. second release liner) from the wound facing surface of the adhesive layer forming part of the wound closure device of the present invention is 0.14 N/40mm. The peel adhesion force for removal of clear polyester release liner (i.e. first release liner) from the non-wound facing surface of the adhesive layer (i.e. strike-through side) forming part of the wound closure device of the present invention is 2.21 N/40mm. In use, the difference in the peel adhesion forces required to remove the paper release liner versus the clear polyester release liner usefully allows for the removal of the paper release liner from the wound facing surface of the adhesive before removal the clear polyester release liner from the non-wound facing surface of the adhesive layer.

It will be appreciated that numerous modifications to the above-described wound closure device and use may be made without departing from the spirit and scope of the invention, for instance, the scope of the invention as defined in the appended claims. Moreover, any one or more of the above-described embodiments could be combined with one or more features of the other embodiments and all such combinations are intended within the present disclosure.

Optional and/or preferred features may be used in other combinations beyond those explicitly described herein and optional and/or preferred features described in relation to one aspect of the invention may also be present in another aspect of the invention, where appropriate.

It should be understood that while the use of words such as "preferable", "preferably", "preferred" or "more preferred" in the description suggest that a feature so described may be desirable, it may nevertheless not be necessary and embodiments lacking such a feature may be contemplated as within the scope of the invention as defined in the appended claims. In relation to the claims, it is intended that when words such as "a," "an," or "at least one," are used to preface a feature there is no intention to limit the claim to only one such feature unless specifically stated to the contrary in the claim.

## Claims

1. A wound closure device comprising:
a wound closing layer comprising a wound facing surface, a non-wound facing surface opposite to the wound facing surface of the wound closing layer and one or more open areas extending through the wound closing layer from the wound facing surface to the non-wound facing surface of the wound closing layer; and
an adhesive disposed on the wound facing surface of the wound closing layer and extending through one or more of the open areas to the extent that, in use, a substrate layer positioned adjacent to the non-wound facing surface of the wound closing layer contacts the adhesive extending through one or more of the open areas.

2. The wound closure device according to claim 1, wherein the substrate layer is positioned adjacent to the non-wound facing surface of the wound closing layer and contacts the adhesive extending through one or more of the open areas.

3. The wound closure device according to claims 1 or 2, wherein:
(i) the adhesive extends through the one or more open areas but does not protrude from the non-wound facing surface of the wound closing layer; or
(ii) the adhesive extends through the one or more open areas and protrudes from the non-wound facing surface of the wound closing layer but without covering the non-wound facing surface of the wound closing layer.

4. The wound closure device according to claims 1-3, wherein the adhesive is an adhesive layer comprising a wound facing surface and a non-wound facing surface opposite to the wound facing surface of the adhesive layer, wherein the substrate layer positioned adjacent to the non-wound facing surface of the wound closing layer contacts the non-wound facing surface of the adhesive layer; optionally wherein the non-wound facing surface of the adhesive layer has a peel adhesion value which is less than the peel adhesion value of a wound facing surface of the adhesive layer.

5. The wound closure device according to claim 4, wherein the peel adhesion value of the non-wound facing surface of the adhesive layer is from about 0.1 N/25mm to about 2.0 N/25mm, preferably, from about, 0.5 N/25mm to about 1.0 N/25mm.

6. The wound closure device according to any one of claims 4 or 5, wherein the peel adhesion value of the wound facing surface of the adhesive layer is about 2.5 N/25mm to about 10 N/25mm, preferably, about 2.5 N/25mm to about 5.0 N/25mm.

7. The wound closure device according to any one of the preceding claims, wherein the wound closing layer has a weight of from about 5 grams per m² to 30 grams per m², preferably, from about 10 grams per m² to 20 grams per m²

8. The wound closure device according to any one of the preceding claims, wherein the wound closing layer is formed of a polymer; optionally wherein the polymer is selected from the group consisting of polyesters, polyolefins and polyamides.

9. The wound closure device according to any one of the preceding claims, wherein the wound closing layer has an open area percentage (%) of from about 50 to about 95, preferably, from about 70 to about 95.

10. The wound closure device according to any one of the preceding claims, wherein the wound closing layer is a mesh or perforated film, preferably, wherein the wound closing layer is a mesh; optionally wherein the mesh is a mesh fabric; optionally wherein the mesh fabric is a woven mesh fabric.

11. The wound closure device according to any one of the preceding claims, wherein:
(i) the adhesive is disposed at a coat weight of from about 5 grams per m² to about 100 grams per m², preferably, the adhesive has coat weight of from about 10 grams per m² to about 30 grams per m²; and / or
(ii) the adhesive comprises a pressure sensitive adhesive, preferably, wherein the adhesive comprises an acrylic based pressure sensitive adhesives or silicone pressure sensitive adhesives or mixture thereof.

12. The wound closure device according to any one of claims 4-11, wherein the substrate layer is a first release liner and there is a second release liner in contact with the wound facing surface of the adhesive layer; optionally wherein the peel adhesion value required to remove the first release liner from the non-wound facing surface of the adhesive layer is greater than the peel adhesion value required to remove the second release liner from the wound facing surface of the adhesive layer.

13. A method of manufacturing a wound closure device comprising:
providing a wound closing layer comprising a wound facing surface, a non-wound facing surface opposite to the wound facing surface and one or more open areas extending through the wound closing layer from the wound facing surface to the non-wound facing surface of the wound closing layer;
disposing an adhesive on the wound facing surface of the wound closing layer and extending through one or more of the open areas to the extent that, in use, a substrate layer positioned adjacent to the non-wound facing surface of the wound closing layer contacts the adhesive extending through one or more of the open areas; optionally wherein the disposing comprises compressing the wound closing layer and the adhesive together at a pressure of from about 20 Psi to about 40 Psi.

14. The method of manufacturing according to claim 13, wherein, prior to the step of disposing, the following steps are performed:
providing a carrier layer comprising a wound facing surface and a non-wound facing surface opposite to the wound facing surface of the carrier layer;
applying an adhesive-solvent mixture onto the non-wound facing surface of the carrier layer; and removing the solvent to form the adhesive; optionally wherein removing the solvent comprises drying at from about 50°C to about 150°C to form the adhesive ; optionally
wherein the adhesive is an adhesive layer comprising a wound facing surface and a non-wound facing surface opposite to the wound facing surface of the adhesive layer, wherein the non-wound facing surface of the carrier layer is in contact with the wound facing surface of the adhesive layer; and the disposing an adhesive comprises:
contacting the wound facing surface of the wound closing layer with the non-wound facing surface of the adhesive layer..

15. A kit of parts for closing a wound comprising:
a wound closure device according to any one of claims 1 to 12; and
an applicator comprising a wound closing adhesive.

## Patentansprüche

1. Wundverschlussvorrichtung, umfassend:
eine wundverschließende Schicht, die eine der Wunde zugewandte Oberfläche, eine der Wunde nicht zugewandte Oberfläche gegenüber jener der Wunde zugewandten Oberfläche der wundverschließenden Schicht und einen oder mehrere offene Bereiche umfasst, die sich durch die wundverschließende Schicht hindurch von der Wunde zugewandten Oberfläche zur nicht der Wunde zugewandten Oberfläche der wundverschließenden Schicht erstrecken; und
einen Klebstoff, der auf der der Wunde zugewandten Oberfläche der wundverschließenden Schicht aufgebracht ist und sich in dem Maße durch einen oder mehrere der offenen Bereiche hindurch erstreckt, dass im Gebrauch eine Substratschicht, die an die nicht der Wunde zugewandten Oberfläche der wundverschließenden Schicht angrenzend positioniert ist, den Klebstoff, der sich durch einen oder mehrere der offenen Bereiche hindurch erstreckt, berührt.

2. Wundverschlussvorrichtung nach Anspruch 1, wobei die Substratschicht an die nicht der Wunde zugewandten Oberfläche der wundverschließenden Schicht angrenzend positioniert ist und den Klebstoff, der sich durch einen oder mehrere der offenen Bereiche hindurch erstreckt, berührt.

3. Wundverschlussvorrichtung nach den Ansprüchen 1 oder 2, wobei:
(i) der Klebstoff sich durch den einen oder mehrere offene Bereiche hindurch erstreckt, jedoch nicht von der nicht der Wunde zugewandten Oberfläche der wundverschließenden Schicht hervorsteht; oder
(ii) der Klebstoff sich durch den einen oder mehrere offene Bereiche erstreckt und von der nicht der Wunde zugewandten Oberfläche der wundverschließenden Schicht hervorsteht, ohne jedoch die nicht der Wunde zugewandte Oberfläche der wundverschließenden Schicht zu bedecken.

4. Wundverschlussvorrichtung nach den Ansprüchen 1-3, wobei der Klebstoff eine Klebstoffschicht ist, die eine der Wunde zugewandte Oberfläche und eine der Wunde nicht zugewandte Oberfläche gegenüber jener der Wunde zugewandten Oberfläche der Klebstoffschicht umfasst, wobei die an die der Wunde nicht zugewandte Oberfläche der wundverschließenden Schicht angrenzend positionierte Substratschicht die der Wunde nicht zugewandte Oberfläche der Klebstoffschicht berührt; wobei die der Wunde nicht gewandte Oberfläche der Klebstoffschicht optional einen Abziehhaftungswert aufweist, der kleiner ist als der Abziehhaftungswert einer der Wunde zugewandten Oberfläche der Klebstoffschicht.

5. Wundverschlussvorrichtung nach Anspruch 4, wobei der Abziehhaftungswert der der Wunde nicht zugewandten Oberfläche der Klebstoffschicht etwa 0,1 N/25 mm bis etwa 2,0 N/25 mm, vorzugsweise etwa 0,5 N/25 mm bis etwa 1,0 N/25 mm beträgt.

6. Wundverschlussvorrichtung nach einem der Ansprüche 4 oder 5, wobei der Abziehhaftungswert der der Wunde zugewandten Oberfläche der Klebstoffschicht etwa 2,5 N/25 mm bis etwa 10 N/25 mm, vorzugsweise etwa 2,5 N/25 mm bis etwa 5,0 N/25 mm beträgt.

7. Wundverschlussvorrichtung nach einem der vorstehenden Ansprüche, wobei die wundverschließende Schicht ein Gewicht von etwa 5 Gramm pro m² bis 30 Gramm pro m², vorzugsweise von etwa 10 Gramm pro m² bis 20 Gramm pro m² aufweist.

8. Wundverschlussvorrichtung nach einem der vorstehenden Ansprüche, wobei die wundverschließende Schicht aus einem Polymer gebildet ist; wobei das Polymer optional aus der Gruppe bestehend aus Polyestern, Polyolefinen und Polyamiden ausgewählt ist.

9. Wundverschlussvorrichtung nach einem der vorstehenden Ansprüche, wobei die wundverschließende Schicht einen Prozentsatz eines offenen Bereichs (%) von etwa 50 bis etwa 95, vorzugsweise von etwa 70 bis etwa 95 aufweist.

10. Wundverschlussvorrichtung nach einem der vorstehenden Ansprüche, wobei die wundverschließende Schicht ein Netz oder ein perforierter Film ist, wobei die wundverschließende Schicht vorzugsweise ein Netz ist; wobei das Netz optional ein Netzgewebe ist; wobei das Netzgewebe optional ein gewebtes Netzgewebe ist.

11. Wundverschlussvorrichtung nach einem der vorstehenden Ansprüche, wobei:
(i) der Klebstoff in einem Beschichtungsgewicht von etwa 5 Gramm pro m² bis etwa 100 Gramm pro m² aufgebracht ist, der Klebstoff vorzugsweise ein Beschichtungsgewicht von etwa 10 Gramm pro m² bis etwa 30 Gramm pro m² aufweist; und/oder
(ii) der Klebstoff einen druckempfindlichen Klebstoff umfasst, wobei der Klebstoff vorzugsweise einen druckempfindlichen Klebstoff auf Acrylbasis oder einen druckempfindlichen Silikonklebstoff oder eine Mischung davon umfasst.

12. Wundverschlussvorrichtung nach einem der Ansprüche 4-11, wobei die Substratschicht eine erste Trennlage ist und eine zweite Trennlage, die mit der der Wunde zugewandten Oberfläche der Klebstoffschicht in Berührung steht, vorhanden ist; wobei der Abziehhaftungswert, der erforderlich ist, um die erste Trennlage von der der Wunde nicht zugewandten Oberfläche der Klebeschicht zu entfernen, optional größer ist als der Abziehhaftungswert, der erforderlich ist, um die zweite Trennlage von der der Wunde zugewandten Oberfläche der Klebeschicht zu entfernen.

13. Verfahren zum Herstellen einer Wundverschlussvorrichtung, umfassend:
Bereitstellen einer wundverschließenden Schicht, die eine der Wunde zugewandte Oberfläche, eine der Wunde nicht zugewandte Oberfläche gegenüber jener der Wunde zugewandten Oberfläche und einen oder mehrere offene Bereiche umfasst, die sich durch die wundverschließende Schicht hindurch von der Wunde zugewandten Oberfläche zur nicht der Wunde zugewandten Oberfläche der wundverschließenden Schicht erstrecken;
Aufbringen eines Klebstoffes auf der der Wunde zugewandten Oberfläche der wundverschließenden Schicht und der sich in dem Maße durch einen oder mehrere der offenen Bereiche hindurch erstreckt, dass im Gebrauch eine Substratschicht, die an die nicht der Wunde zugewandten Oberfläche der wundverschließenden Schicht angrenzend positioniert ist, den Klebstoff, der sich durch einen oder mehrere der offenen Bereiche hindurch erstreckt, berührt; wobei das Aufbringen optional das Verpressen der wundverschließenden Schicht und des Klebstoffs mit einem Druck von etwa 20 Psi bis etwa 40 Psi umfasst.

14. Verfahren zum Herstellen nach Anspruch 13, wobei vor dem Schritt des Aufbringens die folgenden Schritte durchgeführt werden:
Bereitstellen einer Trägerschicht, die eine der Wunde zugewandte Oberfläche und eine der Wunde nicht zugewandten Oberfläche der Trägerschicht gegenüber der der Wunde zugewandten Oberfläche umfasst;
Auftragen eines Klebstoff-Lösungsmittel-Gemisches auf die nicht der Wunde zugewandte Oberfläche der Trägerschicht; und Entfernen des Lösungsmittels, um den Klebstoff zu bilden; wobei das Entfernen des Lösungsmittels optional Trocknen bei etwa 50 °C bis etwa 150 °C umfasst, um den Klebstoff zu bilden; optional
der Klebstoff eine Klebstoffschicht ist, die eine der Wunde zugewandte Oberfläche und eine der Wunde nicht zugewandte Oberfläche der Klebstoffschicht gegenüber der der Wunde nicht zugewandten Oberfläche umfasst, wobei die der Wunde nicht zugewandte Oberfläche der Trägerschicht mit der der Wunde zugewandten Oberfläche der Klebstoffschicht in Berührung steht; und das Aufbringen eines Klebstoffs umfasst:
In-Berührung-bringen der der Wunde zugewandten Oberfläche der wundverschließenden Schicht mit der der Wunde nicht zugewandten Oberfläche der Klebstoffschicht.

15. Teileset zum Verschließen einer Wunde, umfassend:
eine Wundverschlussvorrichtung nach einem der Ansprüche 1 bis 12; und
einen Applikator, der einen wundverschließenden Klebstoff umfasst.

## Revendications

1. Dispositif de fermeture de plaie comprenant :
une couche de fermeture de plaie comprenant une surface tournée vers la plaie, une surface non tournée vers la plaie opposée à la surface tournée vers la plaie de la couche de fermeture de plaie et une ou plusieurs zones ouvertes s'étendant à travers la couche de fermeture de plaie de la surface tournée vers la plaie à la surface non tournée vers la plaie de la couche de fermeture de plaie ; et
un adhésif disposé sur la surface tournée vers la plaie de la couche de fermeture de plaie et s'étendant à travers une ou plusieurs des zones ouvertes dans la mesure où, en utilisation, une couche de substrat positionnée de manière adjacente à la surface non tournée vers la plaie de la couche de fermeture de plaie est en contact avec l'adhésif s'étendant à travers une ou plusieurs des zones ouvertes.

2. Dispositif de fermeture de plaie selon la revendication 1, dans lequel la couche de substrat est positionnée de manière adjacente à la surface non tournée vers la plaie de la couche de fermeture de plaie et est en contact avec l'adhésif s'étendant à travers une ou plusieurs des zones ouvertes.

3. Dispositif de fermeture de plaie selon les revendications 1 ou 2, dans lequel :
(i) l'adhésif s'étend à travers les une ou plusieurs zones ouvertes mais ne fait pas saillie depuis la surface non tournée vers la plaie de la couche de fermeture de plaie ; ou
(ii) l'adhésif s'étend à travers les une ou plusieurs zones ouvertes et fait saillie de la surface non tournée vers la plaie de la couche de fermeture de plaie, mais sans recouvrir la surface non tournée vers la plaie de la couche de fermeture de plaie.

4. Dispositif de fermeture de plaie selon les revendications 1-3, dans lequel l'adhésif est une couche adhésive comprenant une surface tournée vers la plaie et une surface non tournée vers la plaie opposée à la surface tournée vers la plaie de la couche adhésive, dans lequel la couche de substrat positionnée de manière adjacente à la surface non tournée vers la plaie de la couche de fermeture de plaie est en contact avec la surface non tournée vers la plaie de la couche adhésive ; éventuellement dans lequel la surface non tournée vers la plaie de la couche adhésive présente une valeur d'adhérence mesurée par pelage qui est inférieure à la valeur d'adhérence mesurée par pelage d'une surface tournée vers la plaie de la couche adhésive.

5. Dispositif de fermeture de plaie selon la revendication 4, dans lequel la valeur d'adhérence mesurée par pelage de la surface non tournée vers la plaie de la couche adhésive va d'environ 0,1 N/25 mm à environ 2,0 N/25 mm, de préférence, d'environ 0,5 N/25 mm à environ 1,0 N/25 mm.

6. Dispositif de fermeture de plaie selon l'une quelconque des revendications 4 ou 5, dans lequel la valeur d'adhérence mesurée par pelage de la surface tournée vers la plaie de la couche adhésive va d'environ 2,5 N/25 mm à environ 10 N/25 mm, de préférence, d'environ 2,5 N/25 mm à environ 5,0 N/25 mm.

7. Dispositif de fermeture de plaie selon l'une quelconque des revendications précédentes, dans lequel la couche de fermeture de plaie présente un poids d'environ 5 grammes par m² à 30 grammes par m², de préférence d'environ 10 grammes par m² à 20 grammes par m²,

8. Dispositif de fermeture de plaie selon l'une quelconque des revendications précédentes, dans lequel la couche de fermeture de plaie est constituée d'un polymère ; éventuellement dans lequel le polymère est sélectionné parmi le groupe consistant en les polyesters, les polyoléfines et les polyamides.

9. Dispositif de fermeture de plaie selon l'une quelconque des revendications précédentes, dans lequel la couche de fermeture de plaie présente un pourcentage de zones ouvertes (%) d'environ 50 à environ 95, de préférence d'environ 70 à environ 95.

10. Dispositif de fermeture de plaie selon l'une quelconque des revendications précédentes, dans lequel la couche de fermeture de plaie est une maille ou un film perforé, de préférence, dans lequel la couche de fermeture de plaie est une maille ; éventuellement, dans lequel la maille est un tissu à mailles ; éventuellement, dans lequel le tissu à mailles est un tissu à mailles tissé.

11. Dispositif de fermeture de plaie selon l'une quelconque des revendications précédentes, dans lequel :
(i) l'adhésif est disposé selon un poids de couche allant d'environ 5 grammes par m² à environ 100 grammes par m², de préférence, l'adhésif présente un poids de couche allant d'environ 10 grammes par m² à environ 30 grammes par m² ; et/ou
(ii) l'adhésif comprend un adhésif autocollant, de préférence, dans lequel l'adhésif comprend des adhésifs autocollants à base d'acrylique ou des adhésifs autocollants à base de silicone ou un mélange de ceux-ci.

12. Dispositif de fermeture de plaie selon l'une quelconque des revendications 4-11, dans lequel la couche de substrat est un premier revêtement antiadhésif et il existe un second revêtement antiadhésif en contact avec la surface tournée vers la plaie de la couche adhésive ; éventuellement, dans lequel la valeur d'adhérence mesurée par pelage requise pour retirer le premier revêtement antiadhésif de la surface non tournée vers la plaie de la couche adhésive est supérieure à la valeur d'adhérence mesurée par pelage requise pour retirer le second revêtement antiadhésif de la surface tournée vers la plaie de la couche adhésive.

13. Procédé de fabrication d'un dispositif de fermeture de plaie comprenant :
la fourniture d'une couche de fermeture de plaie comprenant une surface tournée vers la plaie, une surface non tournée vers la plaie opposée à la surface tournée vers la plaie et une ou plusieurs zones ouvertes s'étendant à travers la couche de fermeture de plaie de la surface tournée vers la plaie à la surface non tournée vers la plaie de la couche de fermeture de plaie ;
la disposition d'un adhésif sur la surface tournée vers la plaie de la couche de fermeture de plaie et s'étendant à travers une ou plusieurs des zones ouvertes dans la mesure où, en utilisation, une couche de substrat positionnée de manière adjacente à la surface non tournée vers la plaie de la couche de fermeture de plaie est en contact avec l'adhésif s'étendant à travers une ou plusieurs des zones ouvertes ; éventuellement dans lequel la disposition comprend la compression conjointe de la couche de fermeture de plaie et de l'adhésif à une pression allant d'environ 20 Psi à environ 40 Psi.

14. Procédé de fabrication selon la revendication 13, dans lequel, avant l'étape de disposition, les étapes suivantes sont effectuées :
la fourniture d'une couche porteuse comprenant une surface tournée vers la plaie et une surface non tournée vers la plaie opposée à la surface tournée vers la plaie de la couche porteuse ;
l'application d'un mélange adhésif-solvant sur la surface non tournée vers la plaie de la couche porteuse ; et l'élimination du solvant pour former l'adhésif ; éventuellement dans lequel l'élimination du solvant comprend le séchage à une température d'environ 50 °C à environ 150 °C pour former l'adhésif; éventuellement
dans lequel l'adhésif est une couche adhésive comprenant une surface tournée vers la plaie et une surface non tournée vers la plaie opposée à la surface tournée vers la plaie de la couche adhésive, dans lequel la surface non tournée vers la plaie de la couche porteuse est en contact avec la surface tournée vers la plaie de la couche adhésive ; et la disposition d'un adhésif comprend :
la mise en contact de la surface tournée vers la plaie de la couche de fermeture de plaie avec la surface non tournée vers la plaie de la couche adhésive.

15. Kit de parties pour fermer une plaie comprenant :
un dispositif de fermeture de plaie selon l'une quelconque des revendications 1 à 12 ; et
un applicateur comprenant un adhésif de fermeture de plaie.
